# EUROPEAN PATENT SPECIFICATION

(11) **EP 3 709 889 B1**
(45) Date of publication and mention of the grant of the patent: **19.04.2023**
(21) Application number: 18800187.9
(22) Date of filing: 14.11.2018
(51) Int. Cl.: A61B 8/08, A61B 8/12, A61B 8/00, A61B 18/00, A61N 1/00

(54) **ULTRASOUND TRACKING AND VISUALIZATION**
ULTRASCHALLVERFOLGUNG UND -VISUALISIERUNG
SUIVI ET VISUALISATION ULTRASONORE

(30) Priority: 14.11.2017 EP 17201547
(43) Date of publication of application: 23.09.2020
(73) Proprietor: Koninklijke Philips N.V., 5656 AG Eindhoven (NL)
(72) Inventor: GIJSBERS, Gerardus, Henricus, Maria, 5656 AE Eindhoven (NL); KOLEN, Alexander, Franciscus, 5656 AE Eindhoven (NL); BELT, Harm, Jan, Willem, 5656 AE Eindhoven (NL); HARKS, Godefridus, Antonius, 5656 AE Eindhoven (NL)
(74) Representative: Philips Intellectual Property & Standards
(86) International application number: PCT/EP2018/081143
(87) International publication number: WO 2019/096810

(56) References cited:
- WO-A1-2009/063423
- WO-A1-2011/070477
- WO-A1-2016/059498
- WO-A1-2016/178198
- WO-A2-2009/094646
- CN-B- 103 750 859
- US-A1- 2008 285 824
- US-A1- 2012 029 353

## Description

### FIELD OF THE INVENTION

The invention relates to an ultrasound visualization system for tracking a position of an interventional device based on a stream of live ultrasound images of an ultrasound imaging probe. A computer program product is also disclosed. The invention may be used in the medical ultrasound imaging field, and more specifically in ultrasound-based cardiac imaging. The stream of live ultrasound images may exemplarily be provided by a cardiac imaging probe such as an intra cardiac echo, ICE, probe or a trans esophageal echocardiogram, TEE, probe. The interventional device may for example be an ablation catheter.

### BACKGROUND OF THE INVENTION

Interventional cardiology procedures such as the treatment of cardiac arrhythmias by catheter ablation are increasingly using tracking and mapping techniques to navigate interventional devices within the body. The treatment of atrial fibrillation by catheter ablation is an example of such a procedure. In this, an ablation catheter is used to deliver energy to the cardiac wall to destroy living tissue in order to stop undesired conduction of electric pulses that cause the arrhythmia. Catheter navigation and mapping systems such as Carto, produced by Biosense-Webster, and Ensite, produced by St. Jude Medical are routinely used in this procedure to guide the catheter to the treatment site. These known systems typically use magnetic fields and impedance sensing to respectively navigate the interventional device and to perform cardiac mapping.

A drawback of some cardiac navigation systems is their limited ability to visualize cardiac tissue, particularly the cardiac wall. The known systems typically create a virtual 3D model of the targeted anatomy in which the tracked ablation catheter is graphically visualized. The lack of visualization of the anatomy is often compensated-for by using additional X-ray, typically fluoroscopic, imaging to verify the catheter's location. The poor visualization of soft tissue under X-ray typically requires the physician to undergo extensive training in order to be able to correctly determine the catheter position in the fluoroscopic image. In order improve the visualization of cardiac tissue, ultrasound imaging systems such as 2D intra cardiac echography, ICE, are increasingly being used in these procedures. However it can still be difficult to follow the treatment catheter's movements in the ultrasound image.

US patent 8,303,505 B2 describes an apparatus for image guidance and documentation of medical procedures. One embodiment includes combining small field of view images into a recorded image of with a large field of view and aligning the small field of view real time image with the recorded image through correlation of imaging data. A location and orientation determination system may be used to track the imaging system and provide a starting set of image alignment parameters and/or provide change updates to a set of image alignment parameters, which is then further improved through correlating imaging data. The recorded image may be selected according to real time measurement of a cardiac parameter during an image guided cardiac procedure. WO2016059498 discloses an interventional therapy system comprising at least one controller which is configured to and/or obtains a reference image dataset of an object of interest; segments the reference image dataset to determine peripheral outlines of the object in a plurality of image slices; acquires a current image of the object using an ultrasound probe; selects a peripheral outline of a selected image slice of the plurality of slices of the reference image dataset which is determined to correspond to the current image; and modifies the selected peripheral outline of the image slice of the plurality of slices of the reference image dataset in accordance with at least one deformation vector which may be formed in accordance with elastic deformation constraints. The controller may also be configured to select a cross-section of the reference dataset which is determined to correspond with the current image.

However a need exists for improved anatomical visualization during cardiac, and other interventional medical procedures. There is also a need to reduce the X-ray imaging dose to a patient during such procedures.

### SUMMARY OF THE INVENTION

The invention seeks to improve anatomical visualization during medical procedures. The invention also seeks to reduce the X-ray imaging dose to a patient during such procedures. Further advantages from the described invention will be apparent to the skilled person. Thereto an ultrasound visualization system for tracking a position of an interventional device based on a stream of live ultrasound images of an ultrasound imaging probe, is provided. The ultrasound visualization system comprises at least one processor configured to: i) receive the stream of live ultrasound images; ii) extract from the stream of live ultrasound images a reference image comprising reference image data, the reference image including an anatomical feature; iii) extract from the stream of live ultrasound images a current image comprising current image data, the current image being later in time to the reference image and including at least a portion of the anatomical feature and including at least a portion of the interventional device at a current position; iv) match the at least a portion of the anatomical feature in the current image with the anatomical feature in the reference image to determine a spatial relationship between the current position of the interventional device and the anatomical feature in the reference image; and v) indicate, in the reference image, the current position of the interventional device, based on the determined spatial relationship.

The use of ultrasound imaging in the system improves the visualization of soft tissue. Moreover, since the reference image, or "map", is generated from the stream of live ultrasound images, a benefit of the ultrasound visualization system in relation to known tracking systems that generate an anatomical map in a pre-procedural imaging stage includes the obviation of the need to perform two separate medical procedures. Also, since the map is generated from the live ultrasound image stream, it is more up-to-date than a map that is generated a pre-procedural imaging stage. Moreover, the extracted reference image is inherently static, and so any movement of the ultrasound probe after the generation of this "map" is not translated into a movement of the map. This, in combination with indicating the live position of the interventional device in the static map, allows a user of the system to more easily direct the interventional device to an anatomical feature in the map. Also, the X-ray dose to a patient during such a procedure may also be reduced, or even not required, because the ultrasound reference image provides sufficient positioning information of the interventional device in relation to the anatomical feature.

According to one aspect the field of view of the reference image is extended. In this aspect the reference image corresponds to a first field of view, and the current image corresponds to a second field of view that has as an overlapping portion that overlaps with the first field of view and a non-overlapping portion that does not overlap with the first field of view. The at least one processor is further configured to extend the field of view of the reference image by: i) matching the at least a portion of the anatomical feature in the current image with the anatomical feature in the reference image to establish a spatial relationship between the non-overlapping portion of the second field of view and the first field of view; and ii) adapting the reference image to include at least a portion of the non-overlapping portion of the second field of view based on the established spatial relationship. The extended field of view of the reference image therefore includes more anatomical information. During the medical procedure, movements of the ultrasound transducer, for example from heart contractions or from patient breathing, cause changes in the field of view of the current image as compared to that of the reference image. In this aspect the non-overlapping field of view, is used to extend the map. Improved navigation of the interventional device is provided through the ability to track the interventional device over a larger anatomical region. Moreover, the ability to generate such an extended map from the live images means that a more extensive up-to-date map can be generated during a medical procedure. This provides improved guidance because the interventional device can be more accurately guided to a treatment site.

The at least one processor is further configured to:
i) receive from the interventional device a trigger signal indicative of an activation of the interventional device; and to ii) provide a marker in the reference image corresponding to the position of the interventional device at the time at which the interventional device was activated. In so doing, the reference image, i.e. the map, may be used to record anatomical sites where a medical intervention such as the delivery of a therapeutic treatment or the removal of tissue or a measurement of tissue properties has taken place within the anatomy. In one example this aspect may be used to record ablation positions of an ablation catheter that serves as the interventional device. Again, since the map is static, improved navigation is provided by a user's ability to more clearly see anatomical regions where an ablation has taken place.

According to another aspect the at least one processor is further configured to: i) receive, from a positioning system comprising at least one position sensor disposed on the interventional device, and at least one position sensor disposed on the ultrasound imaging probe, position data corresponding to a current position of the position sensor disposed on the interventional device respective the ultrasound imaging probe; and to ii) indicate, in the reference image, the current position of the interventional device, based further on the current position of the position sensor disposed on the interventional device respective the ultrasound imaging probe. This aspect may be useful in more accurately positioning the interventional device in the reference image, particularly when the interventional device is located toward the edge of the field of view of the ultrasound imaging probe and where image resolution may be degraded.

Further aspects of the invention are described with reference to the appended claims.

### BRIEF DESCRIPTION OF THE DRAWINGS

Fig. 1 illustrates an arrangement 110 that includes an ultrasound visualization system 111.
Fig. 2 illustrates an exemplary application in which ultrasound visualization system 111 is used to image a pulmonary vein ostium 222.
Fig. 3 illustrates an arrangement 310 that includes an ultrasound visualization system 111 wherein the field of view of reference image 118 is extended.

### DETAILED DESCRIPTION OF EMBODIMENTS

In order to illustrate the principles of the present invention an ultrasound visualization system is described with particular reference to a cardiac ablation procedure in which an ICE imaging probe serves as the ultrasound imaging probe, and in which an ablation catheter serves as the interventional device. It is however to be appreciated that the invention also finds application in the wider ultrasound imaging field. The ultrasound imaging probe may alternatively be for example a TEE imaging probe, and the interventional device may for example be a catheter, an ablation catheter, an ablation support catheter, a biopsy device, a guidewire, a filter device, a balloon device, a stent, a mitral clip, a left atrial appendage closure device, an aortic valve, a pacemaker lead, an intravenous line, or a surgical tool.

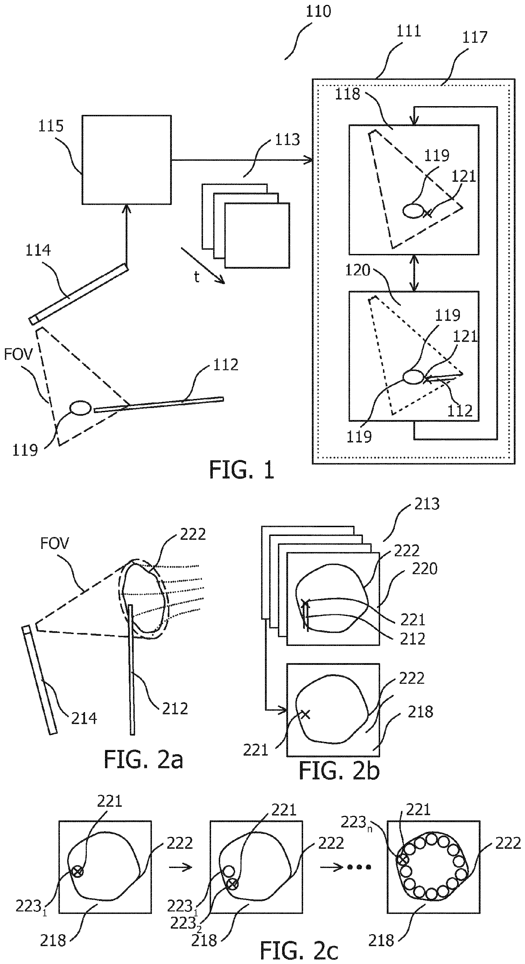

Fig. 1 illustrates an arrangement 110 that includes an ultrasound visualization system 111. Arrangement 110 may be used to track a position of interventional device 112, such as an ablation catheter, based on a stream of live ultrasound images 113 that are provided by an ultrasound imaging probe 114 that is exemplified by an ICE imaging probe having a field of view FOV. Arrangement 110 in Fig. 1 is illustrated as including ultrasound imaging probe console 115 which is configured to receive data from ultrasound imaging probe 114 and to convert said data into a stream of live ultrasound images 113 that are transmitted to ultrasound visualization system 111. Ultrasound imaging probe console 115 may include one or more processors configured to provide this functionality. Ultrasound imaging probe 114 is illustrated as being connected to ultrasound imaging probe console 115, and ultrasound imaging probe console 115 is illustrated as being connected to ultrasound visualization system 111, by means of wired interconnections. However, wireless connections for one or more of these connections are also contemplated. Moreover, it is also contemplated that some or all of the functionality of ultrasound imaging probe console 115 may be included in the ultrasound imaging probe 114 or in ultrasound visualization system 111.

Ultrasound visualization system 111 includes at least one processor 117. As described above, the at least one processor may perform some or all of the processing described in relation to the ultrasound imaging probe console 115. Ultrasound visualization system 111 includes at least one processor 117 that is configured to: i) receive the stream of live ultrasound images 113; ii) extract from the stream of live ultrasound images 113 a reference image 118 comprising reference image data, the reference image including an anatomical feature 119; iii) extract from the stream of live ultrasound images 113 a current image 120 comprising current image data, the current image 120 being later in time to the reference image 118 and including at least a portion of the anatomical feature 119 and including at least a portion of the interventional device 112 at a current position 121; iv) match the at least a portion of the anatomical feature in the current image 120 with the anatomical feature in the reference image 118 to determine a spatial relationship between the current position of the interventional device and the anatomical feature in the reference image 118; and to v) indicate, in the reference image 118, the current position 121 of the interventional device 112, based on the determined spatial relationship. In so doing the current, i.e. live, position of the interventional device is provided in the reference image.

Stream of live ultrasound images 113 may be either 2D or 3D ultrasound images. In a particular example the images are 3D ultrasound images from an ICE imaging probe. Interventional device 112 may in general be any interventional device. In a particular example, interventional device 112 is an ablation catheter. Anatomical feature 119 in reference image 118 may be any anatomical feature that is discernible in an ultrasound image. In the specific example of cardiac ablation it may for example correspond to part of a pulmonary vein ostium. Current image 120 may be in general be any image from the stream of live ultrasound images 113 that is later in time to the reference image. Owing to the live nature of the stream, current image 120 is continually replaced in time by the latest image. Optionally, reference image 218 may be periodically updated. It may be useful to generate a more up-to-date reference image that reflects recent anatomical changes, or for example when the ultrasound probe is moved to a new position of the interventional device within the anatomy.

In one implementation the step of extracting current image 120 from the stream of live ultrasound images 113, may include determining the current position of the interventional device by performing an edge detection method on the current image data to determine an outline of the interventional device. Suitable known edge detection methods include frequency domain filtering and Fourier transformations.

The step of matching the at least a portion of the anatomical feature in the current image 120 with the anatomical feature in the reference image 118 to determine a spatial relationship may be performed by one of many known image processing algorithms. Scale-invariant feature transform, SIFT, is one suitable example. Rigid or non-rigid image transforms known from the medical image processing field may also be used. The correlation technique described in US patent 5,655,535, or the real-time image alignment procedure described in US patent 8,303,505 may alternatively be used. The spatial relationship may be in 2D image space or in 3D image space and may for example be a translation or a rotation or a combination of a rotation and a translation. The spatial relationship may for example be represented as a vector in Cartesian or polar coordinates.

The step of indicating, in the reference image 118, the current position 121 of the interventional device 112, may include for example positioning a marker such as a cross, a circle or other shape, a point, an outline of the interventional device, or locally changing the color, saturation, or hue of a pixel in the reference image.

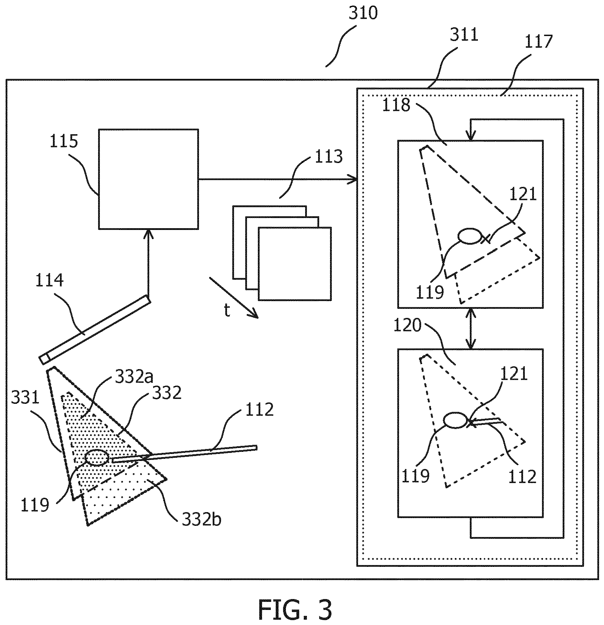

Fig. 2 illustrates an exemplary application in which ultrasound visualization system 111 is used to image a pulmonary vein ostium 222. In Fig. 2a, 3D ICE catheter serving as ultrasound imaging probe 214 is arranged within the heart to view pulmonary vein ostium 222. Interventional device 212, such as a cardiac ablation catheter, is also disposed within the field of view FOV of 3D ICE catheter 214. In Fig. 2b a live stream of ultrasound images 213 of pulmonary vein ostium 222 is illustrated. Reference image 218 is extracted from live stream 213 and includes pulmonary vein ostium 222. Thereafter, reference image 218 serves as a map in which the current, i.e. the most recent, position of the 3D ICE catheter is indicated. The current ultrasound image 220 from live stream 213 includes interventional device 212 at current position 221. Current position 221 of interventional device 212 is subsequently indicated in reference image 218 in the form of exemplary cross 221.

Optionally, reference image 218 in Fig. 2b may be periodically updated. It may be useful to generate a more up-to-date reference image that reflects recent anatomical changes, or for example when the ultrasound probe is moved to a new position within the anatomy.

In one embodiment illustrated in Fig. 2c, interventional device 212 is capable of being activated, and a marker is inserted in reference image 218 at the activation position each time a trigger signal corresponding to the activation is received by the ultrasound visualization system. The marker may be displayed for a time period after the activation in order to provide a record of the activation positions. Continuing with the example in which the interventional device is an ablation catheter, the marker positions, and therefore the trigger signals may correspond to positions at which ablation energy is delivered by the ablation catheter. Thereto, in Fig. 2c the evolution of reference image 218 is illustrated in the case when an ablation catheter serving as interventional device 212 at current positions 221 in pulmonary vein ostium 222 is activated multiple times. Each time interventional device 212 is activated, a marker 223_{1..n} is provided in reference image 218. Marker 223 is illustrated as an exemplary circle and may in general indicate the positions of activation of other interventional devices described herein, such as the activation position of a biopsy device, image positions of a second imaging catheter, tissue measurement positions, the deployment position of a stent activator, and so forth. Other marker shapes may alternatively be used for marker 223.

In operation the functionality described with reference to Fig. 2 may be provided by the system processor described with reference to Fig. 1. Thereto, the processor of Fig. 1 may additionally be configured to: i) receive from the interventional device a trigger signal indicative of an activation of the interventional device; and to ii) provide a marker in the reference image corresponding to the position of the interventional device at the time at which the interventional device was activated.

In one embodiment that is described with reference to Fig. 3, the field of view of reference image 118 may optionally be extended. Fig. 3 illustrates an arrangement 310 that includes an ultrasound visualization system 311 wherein the field of view of reference image 118 is extended. In Fig. 3 reference image 118 corresponds to first field of view 331, and current image 120 corresponds to second field of view 332. Second field of view 332 has an overlapping portion 332a that overlaps with the first field of view 331 and a non-overlapping portion 332b that does not overlap with the first field of view 331. In addition to the functionality described in relation to Fig. 1, processor 117 in Fig. 3 is further configured to extend the field of view of reference image 118 by: i) matching the at least a portion of the anatomical feature 119 in current image 120 with the anatomical feature 119 in reference image 118 to establish a spatial relationship between the non-overlapping portion of the second field of view 332b and the first field of view 331; and ii) adapting reference image 118 to include at least a portion of the non-overlapping portion of the second field of view 332b based on the established spatial relationship.

In Fig. 3 a 2D ultrasound imaging probe 114 is illustrated, however the principles of this embodiment apply equally to a 3D ultrasound imaging probe. Two fields of view 331, 332, are illustrated in Fig. 3. Reference image 118 corresponds to first field of view 331 and current image 120 corresponds to second field of view 332. In Fig. 3, different fields of view 331, 332 are provided by a relative movement between the ultrasound imaging probe and the anatomy. Such a relative movement may be inherent in any medical imaging procedure and caused for example by natural movements of the anatomy, such as contractions of the heart or breathing, or alternatively by a user moving ultrasound imaging probe 114. Different fields of view 331, 332 may also be provided by scanning the field of view of the ultrasound imaging probe. Known beamforming techniques may be used to provide such scanning and may involve adjusting the relative delays between the ultrasound beams transmitted and/or received by ultrasound imaging probe 114. In either situation, second field of view 332 has as an overlapping portion 332a that overlaps with the first field of view 331 and a non-overlapping portion 332b that does not overlap with the first field of view 331.

The same matching procedures as described above in relation to Fig. 1 may be used to establish a spatial relationship between the non-overlapping portion of the second field of view 332b and the first field of view 331. Particular emphasis may be made on the matching procedure at the boundary between the non-overlapping portion of the second field of view 332b and the first field of view 331 in order to provide a seamless transition between the two regions.

The step of adapting the reference image 118 may include using part or all of the image data from the non-overlapping portion of the second field of view 332b to update the reference image, thereby extending it. The adaptation therefore results in an effective field of view that is less than or equal to the combination of fields of views 331, 332 in Fig. 3. Advantageously the extended reference image provides a larger region within which the interventional device can be tracked.

Optionally, in the embodiment of Fig. 3 the step of adapting the reference image may further include using current image data from at least a portion of the overlapping portion of the second field of view 332a to update a corresponding portion of the reference image 118. In other words the reference image may be updated in specific regions in order to reflect recent changes in the anatomy since reference image 118 was extracted from the stream of live ultrasound images. This may be useful for example during an ablation procedure in order to visually determine the extent of an ablation procedure. The updating may for example include averaging the current image data and the reference image data at corresponding positions within the overlapping portion of the second field of view 332a. This averaging may also act so de-noise the reference image, i.e. to improve image quality. Alternatively the reference image data may be entirely replaced by the current image at corresponding positions, or the reference image may be overlaid by the current image at corresponding positions.

In another embodiment, either of the systems described with reference to Fig. 1 or Fig. 3 may be augmented by an additional positioning system that provides position data corresponding to a current position of the position sensor disposed on the interventional device respective the ultrasound imaging probe. This additional position data may be used to improve the positioning accuracy of the system, for example when the interventional device is located in low signal-to-noise ratio region of the current image in the stream of live ultrasound image. The additional positioning system may for example be an ultrasound-based positioning system such as that described in document WO 2011/138698A1 and in the publication "A New Sensor Technology for 2D Ultrasound-Guided Needle Tracking", Huanxiang Lu et al, MICCAI 2014, Part II, LNCS 8674, pp. 389-396, 2014, or a fiberoptic shape-sensing-based positioning system such as that described in US patent 8,050,523 B2, or a magnetic-based positioning system, or an electromagnetic-based positioning system such as that described in US patent 6,233,476 B1. Such positioning systems conventionally employ a position sensor on the interventional device. Thereto, processor 117 of Fig. 1 or Fig. 2 may additionally be configured to: i) receive, from a positioning system comprising at least one position sensor disposed on the interventional device, and at least one position sensor disposed on the ultrasound imaging probe, position data corresponding to a current position of the position sensor disposed on the interventional device respective the ultrasound imaging probe; and to ii) indicate, in the reference image, the current position of the interventional device, based further on the current position of the position sensor disposed on the interventional device respective the ultrasound imaging probe.

One or more of the method steps disclosed herein, particularly those described in relation to the processor of ultrasound visualization system 111, 211 may be recorded in the form of instructions which when executed on a processor cause the processor to carry out such method steps. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray^{™} and DVD.

In summary, an ultrasound visualization system has been described that tracks a position of an interventional device. The system includes a processor that i) receives a stream of live ultrasound images; ii) extracts from the stream of live ultrasound images a reference image comprising reference image data, the reference image including an anatomical feature; iii) extracts from the stream of live ultrasound images a current image comprising current image data, the current image being later in time to the reference image and including at least a portion of the anatomical feature and including at least a portion of the interventional device at a current position; iv) matches the at least a portion of the anatomical feature in the current image with the anatomical feature in the reference image to determine a spatial relationship between the current position of the interventional device and the anatomical feature in the reference image; and v) indicates, in the reference image, the current position of the interventional device, based on the determined spatial relationship.

## Claims

1. Ultrasound visualization system (111, 311) for tracking a position of an interventional device (112) based on a stream of live ultrasound images (113) of an ultrasound imaging probe (114), the ultrasound visualization system (111) comprising at least one processor (117) configured to:
receive the stream of live ultrasound images (113);
extract from the stream of live ultrasound images (113) a reference image (118) comprising reference image data, the reference image including an anatomical feature (119);
extract from the stream of live ultrasound images (113) a current image (120) comprising current image data, the current image (120) being later in time to the reference image (118) and including at least a portion of the anatomical feature (119) and including at least a portion of the interventional device (112) at a current position (121);
match the at least a portion of the anatomical feature in the current image (120) with the anatomical feature in the reference image (118) to determine a spatial relationship between the current position of the interventional device and the anatomical feature in the reference image (118); **characterized in that** the processor is further configured to:
indicate, in the reference image (118), the current position (121) of the interventional device (112), based on the determined spatial relationship;
receive from the interventional device (112) a trigger signal indicative of an activation of the interventional device; and to
provide a marker (223) in the reference image (218) corresponding to the position of the interventional device (112) at the time at which the interventional device was activated.

2. The ultrasound visualization system (311) according to claim 1 wherein the reference image (118) corresponds to a first field of view (331), and wherein the current image (120) corresponds to a second field of view (332) that has as an overlapping portion (332a) that overlaps with the first field of view (331) and a non-overlapping portion (332b) that does not overlap with the first field of view (331); and wherein the at least one processor (117) is further configured to extend the field of view of the reference image (118) by:
matching the at least a portion of the anatomical feature in the current image (120) with the anatomical feature in the reference image (118) to establish a spatial relationship between the non-overlapping portion of the second field of view (332b) and the first field of view (331); and
adapting the reference image (118) to include at least a portion of the non-overlapping portion of the second field of view (332b) based on the established spatial relationship.

3. The ultrasound visualization system according to claim 2 wherein adapting the reference image further comprises using current image data from at least a portion of the overlapping portion of the second field of view (332a) to update a corresponding portion of the reference image (118).

4. The ultrasound visualization system according to claim 3 wherein updating the corresponding portion of the reference image further comprises averaging the current image data and the reference image data at corresponding positions within the overlapping portion of the second field of view (332a).

5. The ultrasound visualization system according to claim 1 wherein extracting the current image (120) from the stream of live ultrasound images (113) further comprises determining the current position (121) of the interventional device by performing an edge detection method on the current image data to determine an outline of the interventional device (112).

6. The ultrasound visualization system according to claim 1 or claim 2 further comprising the ultrasound imaging probe (114) and/ or the interventional device (112).

7. The ultrasound visualization system according to claim 6 wherein the ultrasound imaging probe (114) is an intra cardiac echography, ICE, imaging probe or a trans esophageal, TEE, imaging probe.

8. The ultrasound visualization system according to claim 6 wherein the interventional device (112) is an ablation catheter, an ablation support catheter, or a biopsy device.

9. The ultrasound visualization system according to claim 1 wherein the interventional device is an ablation catheter and wherein the trigger signal is indicative of the delivery of ablation energy by the ablation catheter.

10. The ultrasound visualization system according to any one of claims 1 - 9 wherein the at least one processor (117) is further configured to:
receive, from a positioning system comprising at least one position sensor disposed on the interventional device (112), and at least one position sensor disposed on the ultrasound imaging probe (114), position data corresponding to a current position of the position sensor disposed on the interventional device (112) respective the ultrasound imaging probe (114); and to
indicate, in the reference image (118), the current position (121) of the interventional device, based further on the current position of the position sensor disposed on the interventional device (112) respective the ultrasound imaging probe (114).

11. The ultrasound visualization system according to claim 10 wherein the positioning system is one of: an ultrasound-based positioning system, a fiberoptic shape-sensing-based positioning system, a magnetic-based positioning system, or an electromagnetic-based positioning system.

12. The ultrasound visualization system according to claim 10 further comprising the positioning system.

13. Computer program product comprising instructions which, when executed on a processor (117) of an ultrasound visualization system (111, 311) according to claim 1, cause the processor (117) to carry out the method steps of
receiving the stream of live ultrasound images (113);
extracting from the stream of live ultrasound images a reference image (118) comprising reference image data, the reference image (118) including an anatomical feature (119);
extracting from the stream of live ultrasound images (113) a current image (120) comprising current image data, the current image (120) being later in time to the reference image (118) and including at least a portion of the anatomical feature (119) and including at least a portion of the interventional device (112) at a current position (121);
matching the at least a portion of the anatomical feature (119) in the current image (120) with the anatomical feature (119) in the reference image (118) to determine a spatial relationship between the current position of the interventional device (112) and the anatomical feature in the reference image (118);
indicating, in the reference image (118), the current position (121) of the interventional device (112), based on the determined spatial relationship;
receiving from the interventional device (112) a trigger signal indicative of an activation of the interventional device; and
providing a marker (223) in the reference image (218) corresponding to the position of the interventional device (112) at the time at which the interventional device was activated.

## Patentansprüche

1. Ultraschall-Visualisierungssystem (111, 311) zur Verfolgung einer Position einer Interventionsvorrichtung (112) auf der Grundlage eines Stroms von Live-Ultraschallbildern (113) einer Ultraschall-Bildgebungssonde (114), wobei das Ultraschallvisualisierungssystem (111) mindestens einen Prozessor (117) umfasst, der dafür konfiguriert ist:
Den Strom der Live-Ultraschallbilder zu empfangen (113);
aus dem Strom der Live-Ultraschallbilder (113) ein Referenzbild (118) zu extrahieren, mit Referenzbilddaten, wobei das Referenzbild ein anatomisches Merkmal enthält (119);
aus dem Strom der Live-Ultraschallbilder (113) ein aktuelles Bild (120) zu extrahieren, mit aktuellen Bilddaten, wobei das aktuelle Bild (120) zeitlich nach dem Referenzbild (118) liegt, und das mindestens einen Teil des anatomischen Merkmals (119) enthält, und das zumindest einen Teil der Interventionsvorrichtung (112) an einer aktuellen Position (121) enthält;
den mindestens einen Teil des anatomischen Merkmals im aktuellen Bild (120) mit dem anatomischen Merkmal im Referenzbild (118) abzugleichen, um eine räumliche Beziehung zwischen der aktuellen Position der Interventionsvorrichtung und dem anatomischen Merkmal im Referenzbild (118) herzustellen; **dadurch gekennzeichnet, dass** der Prozessor weiterhin dazu konfiguriert ist:
Auf dem Referenzbild (118) die aktuelle Position (121) der Interventionsvorrichtung (112) anzuzeigen, basierend auf der ermittelten räumlichen Beziehung;
von der Interventionsvorrichtung (112) ein Triggersignal zu empfangen, welches eine Aktivierung der Interventionsvorrichtung anzeigt; und um eine Markierung (223) im Referenzbild (218) bereitzustellen, die der Position der Interventionsvorrichtung (112) zu dem Zeitpunkt entspricht, zu dem die Interventionsvorrichtung aktiviert wurde.

2. Ultraschall-Visualisierungssystem (311) nach Anspruch 1, wobei das Referenzbild (118) einem ersten Sichtfeld (331) entspricht, und wobei das aktuelle Bild (120) einem zweiten Sichtfeld (332) entspricht, das einen überlappenden Teil (332a) aufweist, der sich mit dem ersten Sichtfeld (331) überschneidet, und einen nicht überschneidenden Teil (332b), der sich nicht mit dem ersten Sichtfeld (331) überschneidet; und wobei der mindestens eine Prozessor (117) ferner so konfiguriert ist, dass das Sichtfeld des Referenzbildes (118) erweitert wird durch:
Abgleich des mindestens einen Teils des anatomischen Merkmals im aktuellen Bild (120) mit dem anatomischen Merkmal im Referenzbild (118), um eine räumliche Beziehung zwischen dem nicht überlappenden Teil des zweiten Sichtfeldes (332b) und dem erstes Sichtfeld (331) herzustellen; und
Anpassen des Referenzbildes (118), so dass es mindestens einen Teil des nicht überlappenden Teils des zweiten Sichtfeldes (332b) auf der Grundlage der festgelegten räumlichen Beziehung enthält.

3. Ultraschall-Visualisierungssystem nach Anspruch 2, bei dem das Anpassen des Referenzbildes ferner die Verwendung aktueller Bilddaten von mindestens einem Teil des überlappenden Teil des zweiten Sichtfeldes (332a) umfasst, um einen entsprechenden Teil des Referenzbildes (118) zu aktualisieren.

4. Ultraschall-Visualisierungssystem nach Anspruch 3, wobei die Aktualisierung des entsprechenden Teils des Referenzbildes ferner die Mittelwertbildung der aktuellen Bilddaten und die Referenzbilddaten an entsprechenden Positionen innerhalb des überlappenden Abschnitts des zweiten Sichtfeldes (332a) umfasst.

5. Ultraschall-Visualisierungssystem nach Anspruch 1, bei dem die Extraktion des aktuellen Bildes (120) aus dem Strom von Live-Ultraschallbildern (113) ferner das Bestimmen der aktuellen Position (121) der Interventionsvorrichtung durch Ausführen eines Rand-Erkennungsverfahren an den aktuellen Bilddaten umfasst, um einen Umriss der Interventionsvorrichtung (112) zu bestimmen.

6. Das Ultraschall-Visualisierungssystem nach Anspruch 1 oder Anspruch 2, ferner die Ultraschall-Bildgebungssonde (114) und/oder die Interventionsvorrichtung (112) umfassend.

7. Ultraschall-Visualisierungssystem nach Anspruch 6, wobei die Ultraschall-Bildgebungssonde (114) eine Sonde für die intrakardiale Echographie (ICE) oder eine Trans-Ösophagus, TEE, bildgebende Sonde ist.

8. Ultraschall-Visualisierungssystem nach Anspruch 6, wobei die Interventionsvorrichtung (112) ein Ablationskatheter, ein Ablationsunterstützungskatheter oder ein Biopsiegerät ist.

9. Ultraschall-Visualisierungssystem nach Anspruch 1, wobei die Interventionsvorrichtung ein Ablationskatheter ist und wobei das Triggersignal ein Anzeichen für die Abgabe von Ablationsenergie durch den Ablationskatheter ist.

10. Das Ultraschall-Visualisierungssystem nach einem der Ansprüche 1 bis 9, wobei der mindestens eine Prozessor (117) ferner konfiguriert ist,
von einem Positionierungssystem mit mindestens einem Positionssensor, der auf der Interventionsvorrichtung (112) angeordnet ist und mindestens einen Positionssensor umfasst, der an der Ultraschall-Bildgebungssonde (114) angeordnet ist, Positionsdaten zu empfangen, die einer aktuellen Position des Positionssensors entsprechen, der an der Interventionsvorrichtung (112) bzw. der Ultraschall-Bildgebungssonde (114) angeordnet ist; und
auf dem Referenzbild (118) die aktuelle Position (121) der Interventionsvorrichtung anzuzeigen, basierend auf der aktuellen Position des Positionssensors, der auf der Interventionsvorrichtung (112) bzw. die Ultraschall-Bildgebungssonde (114) angeordnet ist.

11. Ultraschall-Visualisierungssystem nach Anspruch 10, wobei das Positionierungssystem eines der folgenden Systeme ist: Ein ultraschallbasiertes Positionierungssystem, ein faseroptisches Formgebungssystem, ein sensorbasiertes Ortungssystem, ein magnetbasiertes Ortungssystem oder ein elektromagnetisches Ortungssystem.

12. Das Ultraschall-Visualisierungssystem nach Anspruch 10, welches außerdem das Ortungssystem umfasst.

13. Computerprogrammprodukt, das Anweisungen enthält, die bei Ausführung auf einem Prozessor (117) eines Ultraschall-Visualisierungssystems (111, 311) nach Anspruch 1, den Prozessor (117) veranlassen, die folgenden Verfahrensschritte auszuführen:
Empfang des Stroms von Live-Ultraschallbildern (113);
Extraktion eines Referenzbildes aus dem Strom der Live-Ultraschallbilder (118)
mit Referenzbilddaten, wobei das Referenzbild (118) ein anatomisches Merkmal enthält (119);
Extraktion eines aktuellen Bildes (120) aus dem Strom der Live-Ultraschallbilder (113) mit aktuellen Bilddaten, wobei das aktuelle Bild (120) sich zeitlich nach dem Referenzbild (118) befindet, welches mindestens einen Teil des anatomischen Merkmals (119) enthält, und einschließlich mindestens eines Teils der Interventionsvorrichtung (112) an einer aktuellen Position (121);
Abgleich des mindestens einen Teils des anatomischen Merkmals (119) im aktuellen Bild (120) mit dem anatomischen Merkmal (119) im Referenzbild (118), um eine räumliche Beziehung zwischen der aktuellen Position der Interventionsvorrichtung (112) und dem anatomisches Merkmal auf dem Referenzbild (118) herzustellen;
Anzeigen, im Referenzbild (118), der aktuellen Position (121) der Interventionsvorrichtung (112), basierend auf der ermittelten räumlichen Beziehung;
Empfangen eines Auslösesignals von der Interventionsvorrichtung (112), welches eine Aktivierung der Interventionsvorrichtung anzeigt; und
Bereitstellen einer Markierung (223) in dem Referenzbild (218), die der Position der Interventionsvorrichtung (112) entspricht, und zwar zu dem Zeitpunkt, zu dem die Interventionsvorrichtung aktiviert wurde.

## Revendications

1. Un système de visualisation par ultrasons (111, 311) pour le suivi de la position d'un dispositif d'intervention (112) sur la base d'un flux d'images ultrasonores en direct (113) d'une sonde d'imagerie par ultrasons (114), le système de visualisation par ultrasons (111) comprenant au moins un processeur (117) configuré pour:
recevoir le flux d'images ultrasonores en direct (113);
extraire du flux d'images ultrasonores en direct (113) une image de référence (118) comprenant des données d'image de référence, l'image de référence comprenant une caractéristique anatomique (119);
extraire du flux d'images ultrasonores en direct (113) une image courante (120) comprenant des données d'image actuelles, l'image actuelle (120) étant postérieure dans le temps à l'image de référence (118) et comprenant au moins une partie de la caractéristique anatomique (119) et comprenant au moins une partie du dispositif d'intervention (112) à une position actuelle (121);
faire correspondre au moins une partie de la caractéristique anatomique dans l'image actuelle (120) avec la caractéristique anatomique dans l'image de référence (118) afin de déterminer une valeur de référence spatiale entre la position actuelle du dispositif d'intervention et la caractéristique anatomique dans l'image de référence (118); **caractérisée par le fait que** le processeur est également configuré pour:
indiquer, dans l'image de référence (118), la position actuelle (121) du dispositif interventionnel (112), sur la base de la relation spatiale déterminée;
recevoir du dispositif d'intervention (112) un signal de déclenchement indiquant une activation du dispositif d'intervention; et pour
fournir un marqueur (223) dans l'image de référence (218) correspondant à la position du dispositif d'intervention (112) à l'instant où le dispositif d'intervention était activé.

2. Un système de visualisation par ultrasons (311) selon la revendication 1, dans lequel le système (118) correspond à un premier champ de vision (331), et dans lequel l'image (120) correspond à un deuxième champ de vision (332) qui a une partie de chevauchement (332a) qui chevauche le premier champ de vision (331) et une partie sans chevauchement (332b) qui ne chevauche pas le premier champ de vision (331); et dans lequel au moins un processeur (117) est également configuré pour étendre le champ de vision de l'image de référence (118) par:
la mise en correspondance d'au moins une partie de la caractéristique anatomique dans l'image actuelle (120) avec la caractéristique anatomique dans l'image de référence (118) pour établir une relation spatiale entre la partie non chevauchante du second champ de vision (332b) et le premier champ de vision (331); et
l'adaptation de l'image de référence (118) pour inclure au moins une partie de chevauchement du second champ de vision (332b) sur la base de la relation spatiale établie.

3. Un système de visualisation par ultrasons selon la revendication 2, dans lequel l'adaptation de l'image de référence comprend en outre l'utilisation de données d'image actuelles provenant d'au moins une partie de chevauchement du deuxième champ de vision (332a) pour mettre à jour une partie correspondante de l'image de référence (118).

4. Un système de visualisation par ultrasons selon la revendication 3, où la mise à jour de la partie correspondante de l'image de référence comprend en outre le calcul de la moyenne de données d'image actuelle et les données d'images de référence à des positions correspondantes dans la partie de chevauchement du second champ de vision (332a).

5. Un système de visualisation par ultrasons selon la revendication 1, dans lequel l'extraction de l'image actuelle (120) à partir du flux d'images d'échographie en direct (113) comprend en outre la détermination de la position actuelle (121) du dispositif d'intervention en réalisant une méthode de détection des bords sur les données d'image actuelles afin de déterminer un contour du dispositif d'intervention (112).

6. Un système de visualisation par ultrasons selon la revendication 1 ou la revendication 2, comprenant également la sonde d'imagerie à ultrasons (114) et/ou le dispositif d'intervention (112).

7. Un système de visualisation par ultrasons selon la revendication 6, où la sonde d'imagerie ultrasonore (114) est une sonde d'imagerie d'échographie intracardiaque, ICE, ou une sonde d'imagerie transcardiaque oesophagienne, TEE, sonde d'imagerie.

8. Un système de visualisation par ultrasons selon la revendication 6, où le dispositif d'intervention (112) est un cathéter d'ablation, un cathéter de support d'ablation, ou un dispositif de biopsie.

9. Un système de visualisation par ultrasons selon la revendication 1, où le dispositif interventionnel est un cathéter d'ablation et dans lequel le signal de déclenchement est indicatif de la distribution de l'énergie d'ablation par le cathéter d'ablation.

10. Un système de visualisation par ultrasons selon l'une quelconque des revendications 1 à 9 dans lequel au moins un processeur (117) est en outre configuré pour:
recevoir, à partir d'un système de positionnement comprenant au moins un capteur de position disposé sur le dispositif d'intervention (112), et au moins un capteur de position disposé sur le dispositif d'intervention une sonde d'imagerie à ultrasons (114), des données de position correspondant à une position actuelle du capteur de position disposé sur le dispositif d'intervention (112), respectivement la sonde d'imagerie ultrasonore (114); et pour
indiquer, dans l'image de référence (118), la position actuelle (121) du dispositif d'intervention, en se fondant en outre sur la position actuelle du capteur de position disposé sur le dispositif d'intervention (112) respectivement la sonde d'imagerie ultrasonore (114).

11. Un système de visualisation par ultrasons selon la revendication 10, dans lequel le système de positionnement est l'un des systèmes suivants:
un système de positionnement à base d'ultrasons, un système de positionnement à base de fibres optiques ou un système de positionnement fondé sur le magnétisme, ou un système de positionnement fondé sur l'électromagnétisme.

12. Un système de visualisation par ultrasons selon la revendication 10, comprenant en outre le système de positionnement.

13. Un produit de programme informatique comprenant des instructions qui, lorsqu'elles sont exécutées sur un processeur (117) d'un système de visualisation à ultrasons (111, 311) selon la revendication 1, font en sorte que le processeur (117) exécute les étapes de procédé suivantes:
recevoir le flux d'images ultrasonores en direct (113);
extraire du flux d'images ultrasonores en direct une image de référence (118) comprenant des données d'image de référence, l'image de référence (118) comprenant une caractéristique anatomique (119);
extraire du flux d'images ultrasonores en direct (113) une image actuelle (120) comprenant des données d'image actuelles, l'image actuelle (120) étant postérieure dans le temps à l'image de référence (118) et comprenant au moins une partie de la caractéristique anatomique (119) et incluant au moins une partie du dispositif d'intervention (112) à une position actuelle (121);
la mise en correspondance d'au moins une partie de l'élément anatomique (119) dans l'image actuelle (120) avec la caractéristique anatomique (119) dans l'image de référence (118) pour déterminer une relation spatiale entre la position actuelle du dispositif d'intervention (112) et la caractéristique anatomique dans l'image de référence (118);
indiquer, dans l'image de référence (118), la position actuelle (121) du dispositif interventionnel (112), sur la base de la relation spatiale déterminée;
la réception, en provenance du dispositif d'intervention (112), d'un signal de déclenchement indiquant l'activation du dispositif d'intervention; et
fournir un marqueur (223) dans l'image de référence (218) correspondant à la position du dispositif d'intervention (112) à l'instant où le dispositif d'intervention était activé.
